# EUROPEAN PATENT SPECIFICATION

(11) **EP 2 967 713 B1**
(45) Date of publication and mention of the grant of the patent: **23.12.2020**
(21) Application number: 14717631.7
(22) Date of filing: 15.03.2014
(51) Int. Cl.: A61B 18/12, A61B 18/14

(54) **SYSTEMS FOR SPECIALIZATION OF NEUROMODULATION TREATMENT**
SYSTEME ZUR SPEZIALISIERUNG EINER NEUROMODULATIONSBEHANDLUNG
SYSTÈMES DE SPÉCIALISATION DU TRAITEMENT PAR NEUROMODULATION

(30) Priority: 15.03.2013 US 201313844618
(43) Date of publication of application: 20.01.2016
(60) Divisional application: 20207212.0
(73) Proprietor: MEDTRONIC ARDIAN LUXEMBOURG S.A.R.L., 2124 Luxembourg (LU)
(72) Inventor: BALLAKUR, Sowmya, Santa Rosa, California 95403 (US)
(74) Representative: Zimmermann & Partner Patentanwälte mbB
(86) International application number: PCT/US2014/029880
(87) International publication number: WO 2014/145164

(56) References cited:
- WO-A1-2006/095171
- US-A1- 2007 129 720
- US-A1- 2012 116 382
- US-A1- 2013 066 220

## Description

### TECHNICAL FIELD

The present disclosure relates to neuromodulation treatment and, more particularly, to devices, systems, and methods for providing evaluation and feedback to an operator of a device providing neuromodulation treatment.

### BACKGROUND

The sympathetic nervous system (SNS) is a primarily involuntary bodily control system typically associated with stress responses. SNS fibers that innervate tissue are present in almost every organ system of the human body and can affect characteristics such as pupil diameter, gut motility, and urinary output. Such regulation can have adaptive utility in maintaining homeostasis or preparing the body for rapid response to environmental factors. Chronic activation of the SNS, however, is a common maladaptive response that can drive the progression of many disease states. Excessive activation of the renal SNS in particular has been identified experimentally and in humans as a likely contributor to the complex pathophysiology of hypertension, states of volume overload (such as heart failure), and progressive renal disease. For example, radiotracer dilution has demonstrated increased renal norepinephrine ("NE") spillover rates in patients with essential hypertension.

Cardio-renal sympathetic nerve hyperactivity can be particularly pronounced in patients with heart failure. For example, an exaggerated NE overflow from the heart and kidneys is often found in these patients. Heightened SNS activation commonly characterizes both chronic and end stage renal disease. In patients with end stage renal disease, NE plasma levels above the median have been demonstrated to be predictive of cardiovascular diseases and several causes of death. This is also true for patients suffering from diabetic or contrast nephropathy. Evidence suggests that sensory afferent signals originating from diseased kidneys are major contributors to initiating and sustaining elevated central sympathetic outflow.

Sympathetic nerves innervating the kidneys terminate in the blood vessels, the juxtaglomerular apparatus, and the renal tubules. Stimulation of the renal sympathetic nerves can cause increased renin release, increased sodium (Na⁺) reabsorption, and a reduction of renal blood flow. These neural regulation components of renal function are considerably stimulated in disease states characterized by heightened sympathetic tone and likely contribute to increased blood pressure in hypertensive patients. The reduction of renal blood flow and glomerular filtration rate that result from renal sympathetic efferent stimulation are likely a cornerstone of the loss of renal function in cardio-renal syndrome (i.e., renal dysfunction as a progressive complication of chronic heart failure). Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release), and diuretics (intended to counter the renal sympathetic mediated sodium and water retention). These pharmacologic strategies, however, have significant limitations including limited efficacy, compliance issues, side effects, and others.

US 2012/0116382 A1 relates to catheter apparatuses having multi-electrode arrays for renal neuromodulation and associated systems and methods.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1 is a partially-schematic perspective view illustrating a neuromodulation system including a treatment device configured in accordance with an embodiment of the present technology.
FIG. 2 illustrates modulating renal nerves with the treatment device of FIG. 1 configured in accordance with the present technology.
FIG. 3 is a graph depicting an energy delivery algorithm that may be used in conjunction with the system of FIG. 1 in accordance with an embodiment of the technology.
FIG. 4 is a block diagram illustrating an algorithm for evaluating blood flow velocity in accordance with embodiments of the present technology.
FIG. 5 is a block diagram illustrating an algorithm for providing operator feedback upon occurrence of a high impedance condition in accordance with an embodiment of the present technology.
FIG. 6 is a block diagram illustrating an algorithm for providing operator feedback upon occurrence of cardiac instability in accordance with an embodiment of the present technology.
FIG. 7 is a block diagram illustrating an algorithm for providing operator feedback upon occurrence of respiratory instability in accordance with an embodiment of the present technology.
FIG. 8 is a conceptual diagram illustrating the sympathetic nervous system and how the brain communicates with the body via the sympathetic nervous system.
FIG. 9 is an enlarged anatomical view illustrating nerves innervating a left kidney to form a renal plexus surrounding a left renal artery.
FIGS. 10A and 10B are anatomical and conceptual views, respectively, illustrating a human body including a brain and kidneys and neural efferent and afferent communication between the brain and kidneys.
FIGS. 11A and 11B are anatomic views illustrating, respectively, an arterial vasculature and a venous vasculature of a human.

### DETAILED DESCRIPTION

The present technology is generally directed to devices, systems, and methods for providing useful evaluation and feedback to a clinician or other practitioner performing a procedure, such as electrically- and/or thermally-induced renal neuromodulation (i.e., rendering neural fibers that innervate the kidney inert or inactive or otherwise completely or partially reduced in function). In one embodiment, for example, the feedback relates to pre-neuromodulation parameters and, in particular, to customize power delivery. In some embodiments, one or more operating parameters monitored before and/or during treatment may be analyzed based on defined criteria. Such parameters can include those related to temperature, impedance, heart rate, blood flow, respiratory conditions, patient movement and/or other suitable parameters. Based on this pre-neuromodulation feedback, one or more customized treatment algorithms may be selected that are most likely to provide efficacious neuromodulation to the patient.

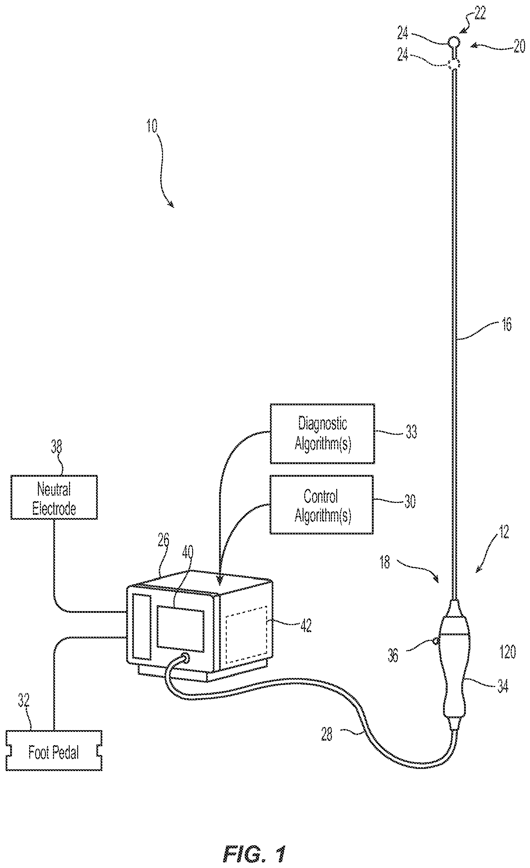

Specific details of several embodiments of the present technology are described herein with reference to FIGS. 1-11B. Although many of the embodiments are described herein with respect to devices, systems, and methods for modulation of renal nerves using electrode-based, transducer-based, cryotherapeutic, direct heat energy, and chemical-based approaches, other applications and other treatment modalities in addition to those described herein are within the scope of the present technology. Additionally, other embodiments of the present technology can have different configurations, components, or procedures than those described herein. For example, other embodiments can include additional elements and features beyond those described herein or be without several of the elements and features shown and described herein. For ease of reference, throughout this disclosure identical reference numbers are used to identify similar or analogous components or features, but the use of the same reference number does not imply that the parts should be construed to be identical. Indeed, in many examples described herein, the identically-numbered parts are distinct in structure and/or function.

As used herein, the terms "distal" and "proximal" define a position or direction with respect to the treating clinician or clinician's control device (e.g., a handle assembly). "Distal" or "distally" can refer to a position distant from or in a direction away from the clinician or clinician's control device. "Proximal" and "proximally" can refer to a position near or in a direction toward the clinician or clinician's control device.

### I. Renal Neuromodulation

Renal neuromodulation is the partial or complete incapacitation or other effective disruption of nerves innervating the kidneys (e.g., rendering neural fibers inert or inactive or otherwise completely or partially reduced in function). For example, renal neuromodulation can include inhibiting, reducing, and/or blocking neural communication along neural fibers (i.e., efferent and/or afferent nerve fibers) innervating the kidneys. Such incapacitation can be long-term (e.g., permanent or for periods of months, years, or decades) or short-term (e.g., for periods of minutes, hours, days, or weeks). Renal neuromodulation is expected to efficaciously treat several clinical conditions characterized by increased overall sympathetic activity, and, in particular, conditions associated with central sympathetic overstimulation such as hypertension, heart failure, acute myocardial infarction, metabolic syndrome, insulin resistance, diabetes, left ventricular hypertrophy, chronic and end stage renal disease, inappropriate fluid retention in heart failure, cardio-renal syndrome, osteoporosis, and sudden death, among others. The reduction of afferent neural signals typically contributes to the systemic reduction of sympathetic tone/drive, and renal neuromodulation is expected to be useful in treating several conditions associated with systemic sympathetic overactivity or hyperactivity. Renal neuromodulation can potentially benefit a variety of organs and bodily structures innervated by sympathetic nerves.

Thermal effects can include both thermal ablation and non-ablative thermal alteration or damage (e.g., via sustained heating and/or resistive heating) to partially or completely disrupt the ability of a nerve to transmit a signal. Desired thermal heating effects, for example, may include raising the temperature of target neural fibers above a desired threshold to achieve non-ablative thermal alteration, or above a higher temperature to achieve ablative thermal alteration. For example, the target temperature can be above body temperature (e.g., approximately 37°C) but less than about 45°C for non-ablative thermal alteration, or the target temperature can be about 45°C or higher for ablative thermal alteration. More specifically, exposure to thermal energy in excess of a body temperature of about 37°C, but below a temperature of about 45°C, may induce thermal alteration via moderate heating of target neural fibers or of vascular structures that perfuse the target fibers. In cases where vascular structures are affected, the target neural fibers may be denied perfusion resulting in necrosis of the neural tissue. For example, this may induce non-ablative thermal alteration in the fibers or structures. Exposure to heat above a temperature of about 45°C, or above about 60°C, may induce thermal alteration via substantial heating of the fibers or structures. For example, such higher temperatures may thermally ablate the target neural fibers or the vascular structures that perfuse the target fibers. In some patients, it may be desirable to achieve temperatures that thermally ablate the target neural fibers or the vascular structures, but that are less than about 90°C, or less than about 85°C, or less than about 80°C, and/or less than about 75°C. Other embodiments can include heating tissue to a variety of other suitable temperatures. Regardless of the type of heat exposure utilized to induce the thermal neuromodulation, a therapeutic effect (e.g., a reduction in renal sympathetic nerve activity (RSNA)) is expected.

Various techniques can be used to partially or completely incapacitate neural pathways, such as those innervating the kidneys. The purposeful application of energy (e.g., RF energy, mechanical energy, acoustic energy, electrical energy, thermal energy, etc.) to tissue and/or the purposeful removal of energy (e.g., thermal energy) from tissue can induce one or more desired thermal heating and/or cooling effects on localized regions of the tissue. The tissue, for example, can be tissue of the renal artery and adjacent regions of the renal plexus, which lay intimately within or adjacent to the adventitia of the renal artery. For example, the purposeful application and/or removal of energy can be used to achieve therapeutically effective neuromodulation along all or a portion of the renal plexus.

### II. Systems and Methods for Renal Neuromodulation

FIG. 1 illustrates a neuromodulation system 10 ("system 10") configured in accordance with an embodiment of the present technology. The system 10 includes an intravascular treatment device 12 operably coupled to a console 26. The treatment device 12 (e.g., catheter) includes an elongated shaft 16 having a proximal portion 18, a handle 34 at a proximal region of the proximal portion 18, and a distal portion 20. The treatment device 12 further includes a therapeutic assembly or treatment section 22 including one or more energy delivery elements 24 (e.g., electrode(s)) at or near the distal portion 20 of the shaft 16. In the illustrated embodiment, a second energy delivery element 24 is illustrated in broken lines to indicate that the systems and methods disclosed herein can be used with treatment devices having one or more energy delivery elements 24. Further, it will be appreciated that although only two energy delivery elements 24 are shown, the treatment device 12 may include additional energy delivery elements 24.

The console 26 can be configured to generate a selected form and/or magnitude of energy for delivery to the target treatment site via the energy delivery element(s) 24. For example, the console 26 can be an energy generator configured to generate radio frequency (RF) energy, pulsed energy, microwave energy, optical energy, ultrasound energy (e.g., intravascularly delivered ultrasound, extracorporeal ultrasound, high-intensity focused ultrasound (HIFU)), direct heat energy, radiation (e.g., infrared, visible, gamma), or another suitable type of energy. In some embodiments, neuromodulation may be achieved by chemical-based treatment including delivering one or more chemicals (e.g., guanethidine, ethanol, phenol, a neurotoxin (e.g., vincristine)), or another suitable agent selected to alter, damage, or disrupt nerves. In a particular embodiment, the console 26 includes an RF generator operably coupled to the one or more energy delivery elements 24 of the therapeutic assembly 22. Furthermore, the console 26 can be configured to control, monitor, supply, or otherwise support operation of the treatment device 12. For example, a control mechanism, such as foot pedal 32, may be connected (e.g., pneumatically connected or electrically connected) to the console 26 to allow an operator to initiate, terminate and/or adjust various operational characteristics of the energy generator, such as power delivery. In other embodiments, the control mechanism may be built into the handle 34.

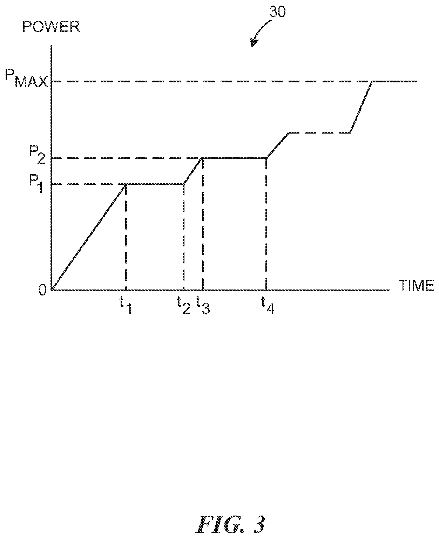

The console 26 can further be configured to deliver neuromodulation energy via one or more automated control algorithms 30 and/or manually under the control of a clinician. The control algorithms 30 can be executed on a processor (not shown) of the system 10 to control the delivery of power and energy to the therapeutic assembly 22. In some embodiments, selection of a control algorithm 30 for a particular patient may be guided by one or more diagnostic algorithms 33 that measure and evaluate one or more operating parameters prior to energy delivery. The diagnostic algorithms 33 provide patient-specific feedback to the clinician which can be used to select an appropriate control algorithm 30 and/or modify the control algorithm 30 to increase the likelihood of efficacious neuromodulation. Further details regarding control and diagnostic algorithms 30 and 33 are described below with reference to FIGS. 3-7.

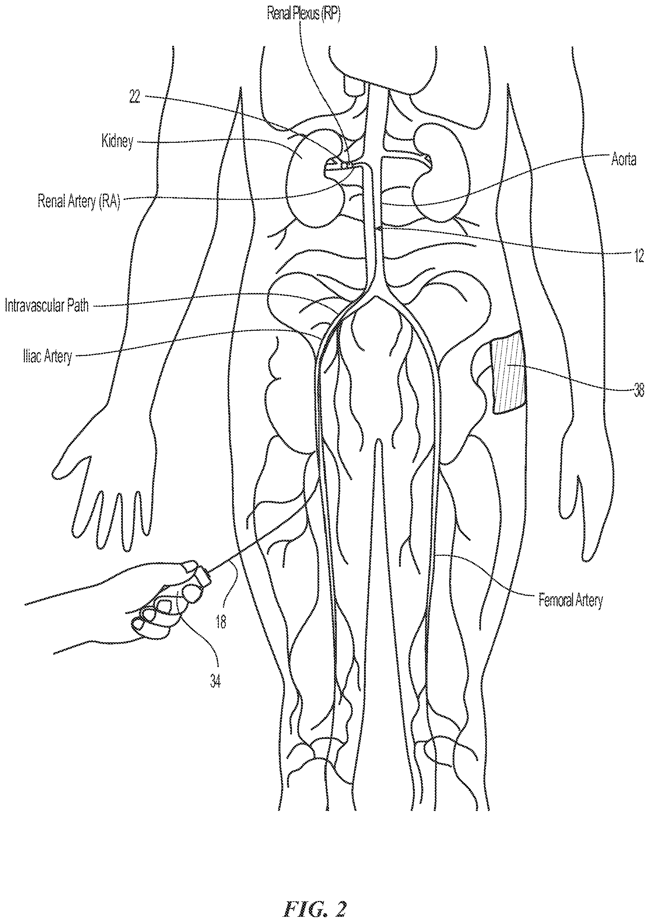

The energy delivery element(s) 24 may be configured to deliver power independently (e.g., may be used in a monopolar fashion), either simultaneously, selectively, or sequentially, and/or may deliver power between any desired combination of the elements (e.g., may be used in a bipolar fashion). In monopolar embodiments, a neutral or dispersive electrode 38 may be electrically connected to the console 26 and attached to the exterior of the patient (e.g., as shown in FIG. 2). Furthermore, the clinician may optionally choose which energy delivery element(s) 24 are used for power delivery in order to form highly customized lesion(s) within the target blood vessel having a variety of shapes or patterns. In still other embodiments, the system 10 can be configured to deliver other suitable forms of treatment energy, such as a combination of monopolar and bipolar electric fields.

The system 10 can further include a controller 42 having, for example, memory (not shown), storage devices (e.g., disk drives), one or more output devices (e.g., a display), one or more input devices (e.g., a keyboard, a touchscreen, etc.) and processing circuitry (not shown). The output devices may be configured to transmit signals to the treatment device 12 (e.g., via the connector 28) to control power to the energy delivery elements 24. In some embodiments the output devices can further be configured to obtain signals from the energy delivery elements 24 and/or any sensors associated with the treatment device 12 such as one or more pressure sensor(s), temperature sensor(s), impedance sensor(s), flow sensor(s), chemical sensor(s), ultrasound sensor(s), optical sensor(s), and other suitable sensing devices. The indicator(s) 40 of the system 10 can serve as one or more output devices and may be a standalone device or may alternatively be associated with the console 26 and/or handle 34. The indicator 40 can include one or more LEDs, a device configured to produce an audible indication, a display screen, and/or other suitable communicative devices. In some embodiments, the indicator can be interactive, such as a user interface that can receive user input and/or provide information to the user and/or processing circuitry for monitoring the one or more sensors. Display devices may be configured to provide indications of power levels or sensor data, such as audio, visual or other indications, or may be configured to communicate the information to another device.

In some embodiments, the controller 42 can be part of the console 26, as shown in FIG. 1. Additionally or alternatively, the controller 42 can be personal computer(s), server computer(s), handheld or laptop device(s), multiprocessor system(s), microprocessor-based system(s), programmable consumer electronic(s), digital camera(s), network PC(s), minicomputer(s), mainframe computer(s), and/or any suitable computing environment. The memory and storage devices are computer-readable storage media that may be encoded with non-transitory, computer-executable instructions (e.g., the control algorithm(s) 30, the feedback algorithm(s) 33, etc.). In addition, the instructions, data structures, and message structures may be stored or transmitted via a data transmission medium, such as a signal on a communications link and may be encrypted. Various communications links may be used, such as the Internet, a local area network, a wide area network, a point-to-point dial-up connection, a cell phone network, Bluetooth, RFID, and other suitable communication channels. The system 10 may be described in the general context of computer-executable instructions, such as program modules, executed by one or more computers or other devices. Generally, program modules include routines, programs, objects, components, data structures, and so on that perform particular tasks or implement particular abstract data types. Typically, the functionality of the program modules may be combined or distributed as desired in various embodiments.

FIG. 2 (with additional reference to FIG. 1) illustrates modulating renal nerves with an embodiment of the system 10. The treatment device 12 provides access to the renal plexus (RP) through an intravascular path, such as from a percutaneous access site in the femoral (illustrated), brachial, radial, or axillary artery to a targeted treatment site within a respective renal artery (RA). As illustrated, a section of the proximal portion 18 of the shaft 16 is exposed externally of the patient. By manipulating the proximal portion 18 of the shaft 16 from outside the intravascular path (e.g., via the handle 34), the clinician may advance the shaft 16 through the sometimes tortuous intravascular path and remotely manipulate or actuate the distal portion 20 of the shaft 16. Image guidance, e.g., computed tomography (CT), fluoroscopy, intravascular ultrasound (IVUS), optical coherence tomography (OCT), or another suitable guidance modality, or combinations thereof, may be used to aid the clinician's manipulation. Further, in some embodiments, image guidance components (e.g., IVUS, OCT) may be incorporated into the treatment device 12 itself. In other embodiments, for example, the treatment device 12 may define a passageway for engaging a guide wire (not shown) for delivery of the therapeutic assembly 22 to the treatment site using over-the-wire ("OTW") or rapid exchange ("RX") techniques.

Once proximity between, alignment with, and contact between the energy delivery element(s) 24 and tissue are established within the respective renal artery (RA), the purposeful application of energy from the console 26 to tissue by the energy delivery element 24 induces one or more desired neuromodulating effects on localized regions of the renal artery (RA) and adjacent regions of the renal plexus (RP), which lay intimately within, adjacent to, or in close proximity to the adventitia of the renal artery. The purposeful application of the energy may achieve neuromodulation along all or a portion of the renal plexus (RP). In some embodiments, the distal portion 20 of the treatment device 12 can be configured to deploy (e.g., expand, bend, deflect, etc.) such that the energy delivery elements 24 contact an inner wall of the renal artery and cause a fully-circumferential lesion without the need for repositioning. For example, the therapeutic assembly 22 can be configured to form a lesion or series of lesions (e.g., a helical/spiral lesion or a discontinuous lesion) that is fully-circumferential overall, but generally non-circumferential at longitudinal segments of the treatment location. This can facilitate precise and efficient treatment with a low possibility of vessel stenosis. In other embodiments, the therapeutic assembly 22 can be configured to form a partially-circumferential lesion or a fully-circumferential lesion at a single longitudinal segment of the treatment location.

A technical objective of a treatment may be, for example, to heat tissue to a depth of at least about 3 mm to a temperature that would lesion a nerve (e.g., about 65°C). A clinical objective of the procedure typically is to neuromodulate (e.g., lesion) a sufficient number of renal nerves (either efferent or afferent nerves of the sympathetic renal plexus) to cause a reduction in sympathetic tone. If the technical objective of a treatment is met (e.g., tissue is heated to about 65°C to a depth of about 3 mm) the probability of forming a lesion of renal nerve tissue is high. The greater the number of technically successful treatments, the greater the probability of modulating a sufficient proportion of renal nerves, and thus the greater the probability of clinical success.

### III. Specialization of Renal Neuromodulation Treatment

### A. Control of Applied Energy

With the treatments disclosed herein for delivering therapy to target tissue, it may be beneficial for energy to be delivered to the target neural structures in a controlled manner. The controlled delivery of energy will allow the zone of thermal treatment to extend into the renal fascia while reducing undesirable energy delivery or thermal effects to the vessel wall. A controlled delivery of energy may also result in a more consistent, predictable and efficient overall treatment. Accordingly, the console 26 desirably includes a processor including a memory component with instructions for executing a control algorithm 30 for controlling the delivery of power and energy to the energy delivery device.

For example, FIG. 3 illustrates one example of a control algorithm 30 configured in accordance with an embodiment of the present technology. When a clinician initiates treatment, the control algorithm 30 includes instructions to the console 26 to gradually adjust its power output to a first power level P₁ (e.g., 5 watts) over a first time period t₁ (e.g., 15 seconds). The power can increase generally linearly during the first time period. As a result, the console 26 increases its power output at a generally constant rate of P₁/t₁. Alternatively, the power may increase exponentially, parabolicly, step-wise, and/or other nonlinear methods. Once P₁ and t₁ are achieved, the algorithm may hold at P₁ until a new time t₂ for a predetermined period of time t₂ - t₁ (e.g., 3 seconds). At t₂ power is increased by a predetermined increment (e.g., 1 watt) to P₂ over a predetermined period of time, t₃ - t₂ (e.g., 1 second). This power ramp in predetermined increments of about 1 watt over predetermined periods of time may continue until a maximum power P_{MAX} is achieved or some other condition is satisfied. In one embodiment, P_{MAX} is 8 watts. In another embodiment P_{MAX} is 6.5 watts. Optionally, the power may be maintained at the maximum power P_{MAX} for a desired period of time or up to the desired total treatment time (e.g., up to about 120 seconds). Although the control algorithm 30 of FIG. 3 comprises a power-control algorithm, it should be understood that the control algorithm 30 additionally or alternatively may include temperature control and/or current control. For example, power may be gradually increased until a desired temperature (or temperatures) is obtained for a desired duration (or durations).

The control algorithm 30 also includes continuously and/or periodically monitoring certain operating parameters such as temperature, time, impedance, power, blood flow velocity, volumetric flow rate, blood pressure, heart rate, and/or other suitable parameters. The control algorithm 30 can also calculate and/or monitor derivatives of such operating parameters, such as temperature over a specified time, a maximum temperature, a maximum average temperature, a minimum temperature, a temperature at a predetermined or calculated time relative to a predetermined or calculated temperature, an average temperature over a specified time, a maximum blood flow, a minimum blood flow, a blood flow at a predetermined or calculated time relative to a predetermined or calculated blood flow, an average blood flow over time, a maximum impedance, a minimum impedance, an impedance at a predetermined or calculated time relative to a predetermined or calculated impedance, a change in impedance over a specified time, a change in impedance relative to a change in temperature over a specified time, and other suitable derivatives. As used herein, "operating parameters" includes operating parameter measurements, derivatives, manipulations, etc. Measurements may be taken at one or more predetermined times, ranges of times, calculated times, and/or times when or relative to when a measured event occurs.

During treatment, the control algorithm 30 checks the monitored operating parameters against predetermined parameter profiles to assess the likelihood of success of the treatment. Generally, it is believed that the greatest probability of less than ideal treatment occurs when an energy delivery element 24 is not in consistent contact with the vessel wall. Accordingly, the control algorithm 30 can adjust and/or terminate treatment when one or more operating parameters fall outside of a pre-determined range, thereby indicating inconsistent or poor contact between an energy delivery element 24 and the vessel wall.. For example, if the monitored parameters fall within the ranges set by the predetermined parameter profiles, then treatment may continue at the commanded power output. If monitored parameters fall outside the ranges set by the predetermined parameter profiles, the control algorithm 30 can adjust and/or terminate/discontinue the commanded power output accordingly so as to increase the likelihood of success of the treatment. In some instances, the control algorithm 30 may cause a message to be displayed, such as on the indicator 40. A message can indicate information such as a type of patient condition (e.g., an abnormal patient condition), the type and/or value of the parameter that falls outside an accepted or expected threshold and/or range, an indication of suggested action for a clinician, or an indication that energy delivery has been stopped. However, if no unexpected or aberrant measurements are observed, energy may continue to be delivered at the target site in accordance with the pre-selected control algorithm 30 for a specified duration resulting in a complete treatment.

Temperature is one example of an operating parameter that may be monitored and checked against predetermined parameters by the control algorithm 30 during treatment. A temperature rise in the treatment energy delivery element 24 is a result of heat conducting from tissue to the energy delivery element 24. If an energy delivery element 24 is not in sufficient contact with a vessel wall, energy is delivered into the blood flowing around the energy delivery element 24 and the temperature of the energy delivery element 24 does not increase as expected. Accordingly, a temperature rise during energy delivery below a minimum temperature change threshold (T_{DL}) can indicate insufficient contact between the energy delivery element 24 and the tissue and trigger the control algorithm 30 to modify and/or terminate treatment. Additionally, measured temperatures above a critical temperature threshold T_{C} (e.g., 85°C) could indicate tissue desiccation. As a result, the control algorithm 30 may decrease or stop the power and energy delivery to prevent undesirable thermal effects to target or non-target tissue, such as thrombosis, charring, unreliable lesion size, acute constriction of the artery or a protrusion of the artery wall, and others.

Impedance parameters may also be monitored since impedance values indicate the electrical properties of the treatment site. In thermal inductive embodiments, when an electric field is applied to the treatment site, the impedance will decrease as the tissue cells become less resistive to current flow. If too much energy is applied, tissue desiccation or coagulation may occur near the energy delivery element 24, which increases the impedance as the cells lose water retention and/or the electrode surface area decreases (e.g., via the accumulation of coagulum). Thus, a measured impedance outside of an impedance threshold I_{C} and/or an increase in impedance above a relative threshold I_{R} (e.g., <20 Ohms or >500 Ohms) may be indicative or predictive of undesirable thermal alteration to target or non-target tissue or that a sufficient lesion has been formed and treatment can be stopped.

Furthermore, operating parameters related to power and/or time may trigger adjustment and/or terminate of energy delivery, including: a measured power exceeds a power threshold (e.g., >8 watts or >10 watts), a measured duration of power delivery exceeds a time threshold (e.g., >120 seconds), and/or a set time (e.g., 2 minutes) is checked to prevent indefinite delivery of power.

### B. Selection of Customized Algorithms Based on Pre-Neuromodulation Feedback

Before treatment begins, one or more diagnostic algorithms 33 can detect certain operating parameter(s) which denote a possibility that one or more of the control algorithm(s) 30 will not provide efficacious treatment to the particular patient and/or adequately evaluate patient-specific physiological parameters in response to neuromodulation. Similar to the discussion above with respect to the control algorithm 30, such operating parameters detected by the diagnostic algorithm(s) 33 include impedance, temperature, and/or blood flow parameters that are outside of accepted or expected thresholds and/or predetermined or calculated ranges. Accordingly, evaluation of certain operating parameters by the diagnostic algorithm(s) 33 prior to beginning treatment can inform the clinician as to which control algorithm(s) 30 are most likely to provide successful neuromodulation to the individual patient. The diagnostic algorithm(s) 33 can indicate a particular control algorithm 30 via the indicator 40 based on the patient profile developed by the diagnostic algorithm 33 and/or the diagnostic algorithm 33 can cause the patient profile to be displayed or indicated to the clinician so that the clinician can make an informed selection of the appropriate control algorithm 30 and/or modification of the control algorithm 30. In some instances, the diagnostic algorithm 33 may indicate that the patient is not a good candidate for neuromodulation and the clinician may decide not to pursue treatment.

Predetermined operating parameter thresholds and/or ranges can be empirically determined to create a look-up table. The look-up table may provide change in temperature thresholds and/or ranges for corresponding blood flow velocity values. Other temperature parameters as well as power thresholds and/or ranges and length of treatment time can be similarly determined. Look-up table values can be empirically determined, for example, based on animal studies. In some embodiments, predetermined parameters may be determined based on a particular patient's system response to a small pulse of neuromodulation energy (e.g., RF).

### 1. Pre-Neuromodulation Feedback Related to Blood Flow

Blood flow rates can often be an important operating parameter since blood flow can affect temperature measurements that inform the clinician as to the degree of contact between the energy delivery element 24 and the tissue, as well as the relative success of the treatment. Blood flow rates from patient to patient can vary, and as a result, a standardized control algorithm(s) 30 may not be appropriate across all treatments. For example, if a patient has relatively low blood flow, the energy delivery element 24 temperature increases faster than normal during energy delivery and can reach a predetermined critical temperature (T_{C}) before the tissue can be adequately heated since the control algorithm 30 would prematurely terminate energy delivery at the critical temperature (T_{C}) (discussed above). In other words, under low flow conditions, a standardized control algorithm 30 may terminate treatment before a deep lesion can be created, thus significantly lowering the likelihood of efficacious neuromodulation. If a patient has relatively high blood flow, the blood can pull heat away from the energy delivery element 24 faster than the tissue can heat the energy delivery element 24. In other words, the high blood flow can effectively reduce conductive heating between the tissue and the energy delivery element 24 such that a change in energy delivery element 24 temperature does not meet a minimum temperature change threshold (T_{DL}) during energy delivery (discussed above). Therefore, high blood flow can cause a standardized control algorithm 30 to terminate or reduce treatment since the energy delivery element 24 temperature may not increase as much or as quickly as expected during energy delivery.

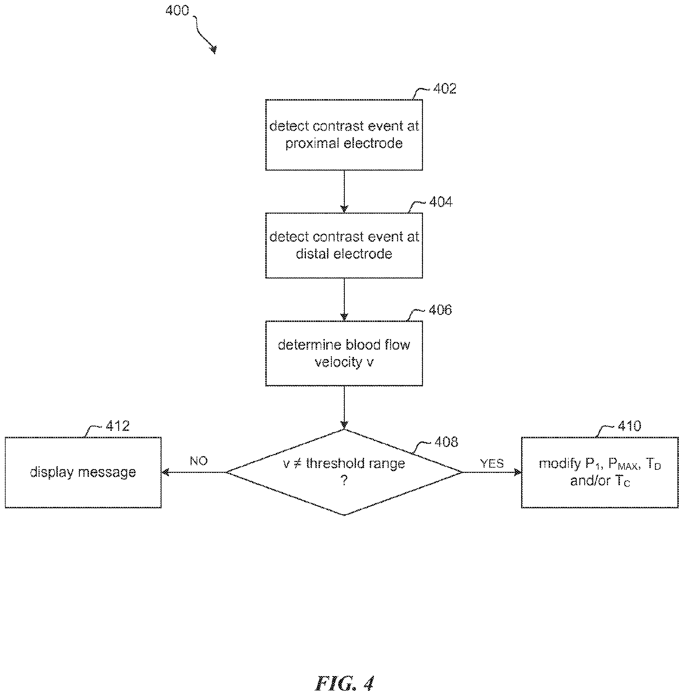

Accordingly, disclosed herein are one or more diagnostic algorithms 33 that determine blood flow velocity prior to initiating treatment and provide feedback to the clinician as to selection and/or modification of the control algorithm 30. For example, FIG. 4 is a block diagram illustrating a diagnostic algorithm 400 for determining blood flow velocity in accordance with an embodiment of the present technology. To facilitate positioning of the therapeutic assembly 22 under fluoroscopic guidance at the treatment site, contrast is injected into the target blood vessel upstream of the energy delivery element(s) 24 before initiation of energy delivery. Breach of one or more of the energy delivery elements 24 by the injected contrast causes both an increase in impedance and a corresponding drop in temperature (referred to herein as a "contrast event"). The diagnostic algorithm 400 monitors impedance and temperature parameters. When the algorithm 400 detects an increase in impedance above a predetermined threshold, the algorithm 400 can evaluate temperature measurements during a corresponding time period for one or more temperature parameters that indicate a decrease in temperature (e.g., decreasing average temperature, negative rate of change of temperature, etc.). If this decrease in temperature is detected along with the increase in impedance, the diagnostic algorithm 33 detects a contrast event.

To determine blood flow velocity, the algorithm 400 detects a contrast event first at a proximal energy delivery element (block 402) and then at a distal energy delivery element (block 404). In some embodiments, for example, the proximal energy delivery element and the distal energy delivery element can be fixedly positioned along the shaft 16 relative to each other such that a separation distance D between the proximal delivery element and the distal delivery element along the length of the shaft 16 is generally constant and can be measured prior to intravascular delivery. In some embodiments, such as when the therapeutic element 22 has an expandable helical/spiral configuration, the therapeutic element 22 can be positioned at the treatment site and the separation distance D can be measured from a fluoroscopic image. The separation distance D can then be manually entered via a user interface on the console 26. In some embodiments, the separation distance D could additionally or alternatively be measured from an angiograph taken at the treatment site. Imaging processing techniques may also be utilized to automatically calculate separation distance D and input this value to the controller 42 for use by the algorithm 400.

Furthermore, the separation distance D between the energy delivery elements 24 could be determined based on the inner diameter of the targeted vessel (e.g., a renal artery). Because the therapeutic element 22 expands to be in apposition with the vessel wall, the vessel inner diameter may correspond to a certain known spacing between energy delivery elements 24. As mentioned above, the vessel inner diameter could be manually measured via various imaging techniques, such as angiography. Likewise, the inner diameter of the vessel wall can be automatically determined using image processing of the angiograph that calculates an inner diameter value and inputs this value directly to the controller 42. Additionally, the therapeutic element 22 may include force sensors that, coupled with the controller 42, calculate the shear force on the energy delivery elements 24 and can translate this value to an equivalent vessel diameter value. One or more of these methods may be utilized to determine the separation distance D between the energy delivery elements 24.

Accordingly, the algorithm 400 can determine a blood flow velocity (block 406), for example, by dividing the separation distance D by a difference in time between the two contrast events. The algorithm 400 can evaluate this determined blood flow velocity in view of a predetermined threshold value (decision block 408). If the determined blood flow velocity falls within a predetermined range, the control algorithm 30 is likely to deliver efficient and effective neuromodulation without modification. In this event, a message may be displayed (block 412) instructing the clinician to begin neuromodulation. In some embodiments, the message may additionally include the determined blood flow velocity value.

However, if the determined blood flow velocity is outside of a predetermined range (e.g., higher or lower), then certain control algorithm(s) 30 may be considered inadequate to provide efficacious neuromodulation. As discussed above, the system 10 may be programmed such that during neuromodulation, the control algorithm 30 terminates and/or modifies energy delivery automatically in response to detecting energy delivery element(s) 24 temperatures outside of predetermined thresholds. Accordingly, before initiation of energy delivery, the diagnostic algorithm 400 can select and/or modify the control algorithms 30 to account for variations in blood flow. The following is a non-exhaustive list of actions by which the algorithm 400 may select or suggest a customized control algorithm 30 and/or modify an existing control algorithm 30:
(1) For low blood flow, the algorithm 400 can lower power thresholds (e.g., P₁, P_{MAX}, etc.) of the control algorithm 30.
(2) For low blood flow, the algorithm 400 can modify the timing and/or rate of power delivery (e.g., t₁, t₂... tᵢ).
(3) For high blood flow, the algorithm 400 can lower the increase in temperature change minimum threshold T_{DL}.
(4) For low blood flow, the algorithm 400 can select one or more customized "low-blood flow" control algorithms with power and temperature thresholds that account for low blood flow (described above).
(5) For high blood flow, the algorithm 400 can select one or more customized "high-blood flow" control algorithms with power and temperature thresholds that account for high blood flow (described above).

### 2. Pre-Neuromodulation Feedback Related to Impedance

Like certain temperature parameters, impedance parameters can also inform the clinician as to the degree of contact between the energy delivery element 24 and tissue at the vessel wall, as well as the relative success of the treatment since impedance values indicate the electrical properties of the treatment site. For example, it is expected that impedance will decrease during treatment as tissue is gradually heated as the tissue cells become less reistive to curent flow. However, if the tissue gets too hot it may desicate and its impedance may increase as the cells lose water retention and/or the electrode surface area decreases (e.g., via the accumulation of coagulum). For example, (e.g., detected impedance parameters such as measured impedance outside of an impedance threshold I_{C} and/or an increase in impedance above a relative threshold I_{R} (e.g., <20 Ohms or >500 Ohms) can trigger a standardized control algorithm 30 to terminate treatment. However, impedance parameters in certain situations, such as sudden patient movement, high cardiac conditions, and/or chronic respiration, can exhibit recognizable patterns that, if detected, can inform the clinician of the nature of the instability and/or abnormal impedance data. Accordingly, it may be beneficial to detect such sudden and chronic instabilities prior to initiating energy delivery so that a clinician may reposition at least a portion of the treatment device 12 before beginning neuromodulation.

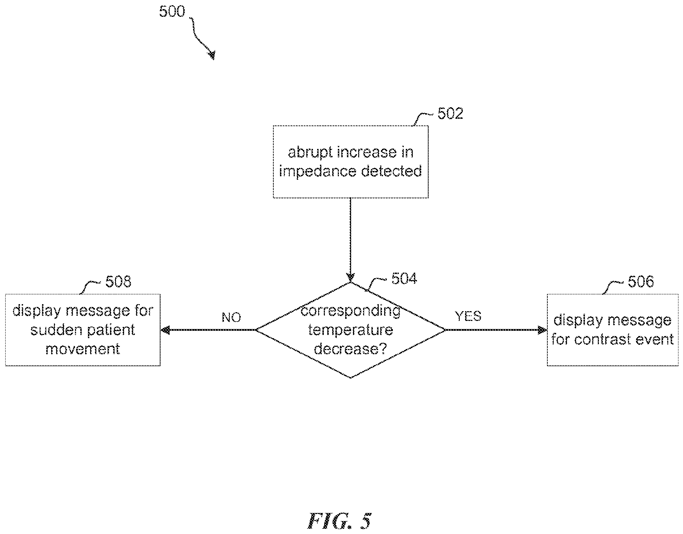

FIG. 5, for example, is a block diagram illustrating a diagnostic algorithm 500 for providing operator feedback upon occurrence of a high impedance condition in accordance with an embodiment of the present technology. In one implementation, the algorithm 500 is executed in response to a spike or abrupt increase in impedance (block 502) and evaluates temperature data (decision block 504) within the time frame corresponding to the spike in impedance to determine if the impedance event was involved in a situation that included sudden instability or if it did not. Sudden instability can be caused, for example, by sudden movement of the patient or catheter, thereby causing the electrode to be pushed harder (i.e., contact force is increased) into the vessel wall, which could also be accompanied by movement to another location. However, a similar spike in impedance can also be detected during a contrast event (as discussed above), which is accompanied by a decrease in temperature. Accordingly, it may be beneficial to characterize the detected impedance event to both notify the clinician of a sudden instability, and also to filter out contrast events that are not indicative of patient movement. In the event that a corresponding temperature decrease is not detected at decision block 504, a first message may be displayed (block 506), such as an indication that a contrast event has been detected. In the event that a corresponding temperature decrease is not detected at decision block 504, an alternative message may be displayed (block 508), such as an indication of sudden patient movement and an instruction to the clinician to reposition the therapeutic assembly 22 and/or establish better tissue contact.

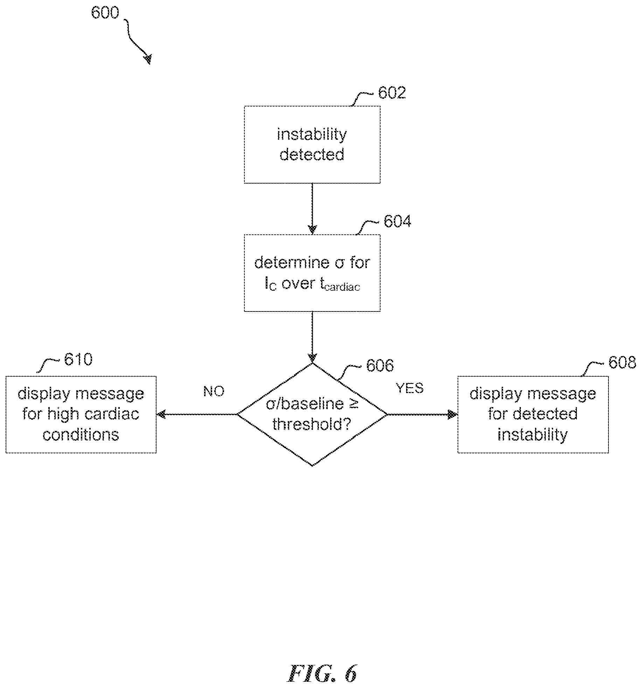

During high cardiac events, impedance may fluctuate in a recognizable pattern as a result of the energy delivery element 24 sliding back and forth across the vessel wall. In some situations, for example, the energy delivery element(s) 24 can be properly positioned within the artery in a consistent manner, but because of axial movement of the vessel (e.g., due to the cardiac event), impedance measurements will vary from the expected impedance data, which typically comprises a sinusoidal wave. In such situations, the variations in impedance measurements can be recognized as an impedance offset consistent with cardiac events. FIG. 6, for example, is a block diagram illustrating another algorithm 600 for providing operator feedback upon occurrence of a high or unstable impedance condition in accordance with an embodiment of the present technology. In one implementation, the algorithm 600 is executed in response to a detected instability (block 602) and evaluates impedance data during a complete cardiac cycle t_{cardiac} (e.g., about 1 to 1.5 seconds) to determine if the impedance event was a result of high cardiac conditions. In some embodiments, evaluation of impedance data during a cardiac cycle can include determining the standard deviation σ_{I} of the impedance measurements taken during a cardiac cycle t_{cardiac} (block 604) and comparing that standard deviation σ_{I} divided by a predetermined baseline value to a predetermined threshold (decision block 606). In the event that the determined impedance statistic is greater than or equal to a predetermined threshold at decision block 606, a first message may be displayed (block 610), such as an indication that high cardiac conditions are detected and/or an instruction to the clinician to reposition the treatment device 12. In the event that the determined impedance statistic is less than a predetermined threshold (at decision block 606), an alternative message may be displayed (block 608), such as an indication of sudden patient movement and an instruction to the clinician to reposition the therapeutic assembly 22 and/or establish better tissue contact.

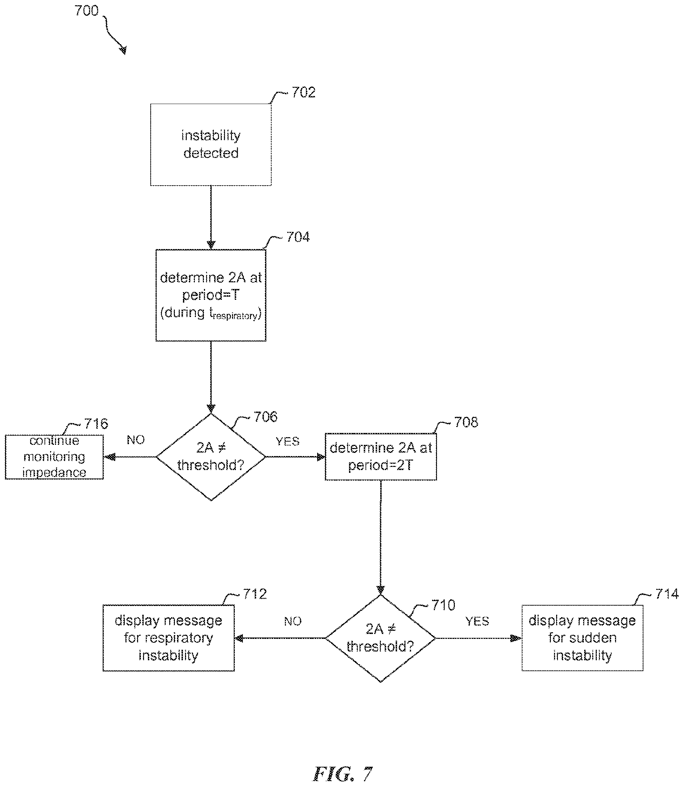

During chronic respiration, impedance may fluctuate in a recognizable pattern as a result of the energy delivery element(s) 24 periodically losing and re-establishing contact with the vessel wall due to the patient's breathing cycle. In such situations, measured impedance can be characteristically and/or uniformly offset from expected impedance measurements, which typically comprise a sinusoidal wave. Respiratory offsets can be distinguished from cardiac offsets since the respiratory cycle is significantly longer than the cardiac cycle. FIG. 7, for example, is a block diagram illustrating yet another diagnostic algorithm 700 for providing operator feedback upon occurrence of a high or unstable impedance condition in accordance with an embodiment of the present technology. In one implementation, the algorithm 700 is executed in response to a detected instability (block 702) and evaluates impedance data during one and a half respiratory cycles t_{respiratory} (e.g., about 10-20 seconds) to determine if the impedance event was a result of chronic respiration. In some embodiments, evaluation of impedance data during a respiration cycle can include determining two times the amplitude A (2A) of the impedance measurements taken during one period (T) of a respiratory cycle t_{respiratory} (block 704) and comparing 2A to a predetermined range (decision block 706). In the event that two times the amplitude 2A is outside of the predetermined range, the algorithm 700 then determines two times the amplitude 2A of the impedance measurements taken during the subsequent respiratory cycle (block 708) and compares 2A again to a predetermined range (decision block 710). If 2A is within of a predetermined threshold at decision block 710, a first message may be displayed (block 712), such as an indication that high cardiac conditions are detected and/or an instruction to the clinician to reposition the treatment device 12. In the event that 2A is outside of a predetermined range at decision block 710, an alternative message may be displayed (block 714), such as an indication of sudden patient movement and an instruction to the clinician to reposition the treatment device 12 and/or establish better tissue contact. In other embodiments, the algorithm 700 may include one or more additional steps and/or one of the steps described above may be modified.

### IV. Pertinent Anatomy and Physiology

The following discussion provides further details regarding pertinent patient anatomy and physiology. This section is intended to supplement and expand upon the previous discussion regarding the relevant anatomy and physiology, and to provide additional context regarding the disclosed technology and the therapeutic benefits associated with renal neuromodulation. For example, as mentioned previously, several properties of the renal vasculature may inform the design of treatment devices and associated methods for achieving renal neuromodulation via intravascular access, and impose specific design requirements for such devices. Specific design requirements may include accessing the renal artery, facilitating stable contact between the energy delivery elements of such devices and a luminal surface or wall of the renal artery, and/or effectively modulating the renal nerves with the neuromodulatory apparatus.

### A. The Sympathetic Nervous System

The SNS is a branch of the autonomic nervous system along with the enteric nervous system and parasympathetic nervous system. It is always active at a basal level (called sympathetic tone) and becomes more active during times of stress. Like other parts of the nervous system, the sympathetic nervous system operates through a series of interconnected neurons. Sympathetic neurons are frequently considered part of the peripheral nervous system (PNS), although many lie within the central nervous system (CNS). Sympathetic neurons of the spinal cord (which is part of the CNS) communicate with peripheral sympathetic neurons via a series of sympathetic ganglia. Within the ganglia, spinal cord sympathetic neurons join peripheral sympathetic neurons through synapses. Spinal cord sympathetic neurons are therefore called presynaptic (or preganglionic) neurons, while peripheral sympathetic neurons are called postsynaptic (or postganglionic) neurons.

At synapses within the sympathetic ganglia, preganglionic sympathetic neurons release acetylcholine, a chemical messenger that binds and activates nicotinic acetylcholine receptors on postganglionic neurons. In response to this stimulus, postganglionic neurons principally release noradrenaline (norepinephrine). Prolonged activation may elicit the release of adrenaline from the adrenal medulla.

Once released, norepinephrine and epinephrine bind adrenergic receptors on peripheral tissues. Binding to adrenergic receptors causes a neuronal and hormonal response. The physiologic manifestations include pupil dilation, increased heart rate, occasional vomiting, and increased blood pressure. Increased sweating is also seen due to binding of cholinergic receptors of the sweat glands.

The sympathetic nervous system is responsible for up- and down-regulating many homeostatic mechanisms in living organisms. Fibers from the SNS innervate tissues in almost every organ system, providing at least some regulatory function to things as diverse as pupil diameter, gut motility, and urinary output. This response is also known as *sympatho-adrenal response* of the body, as the preganglionic sympathetic fibers that end in the adrenal medulla (but also all other sympathetic fibers) secrete acetylcholine, which activates the secretion of adrenaline (epinephrine) and to a lesser extent noradrenaline (norepinephrine). Therefore, this response that acts primarily on the cardiovascular system is mediated directly via impulses transmitted through the sympathetic nervous system and indirectly via catecholamines secreted from the adrenal medulla.

Science typically looks at the SNS as an automatic regulation system, that is, one that operates without the intervention of conscious thought. Some evolutionary theorists suggest that the sympathetic nervous system operated in early organisms to maintain survival as the sympathetic nervous system is responsible for priming the body for action. One example of this priming is in the moments before waking, in which sympathetic outflow spontaneously increases in preparation for action.

### 1. The Sympathetic Chain

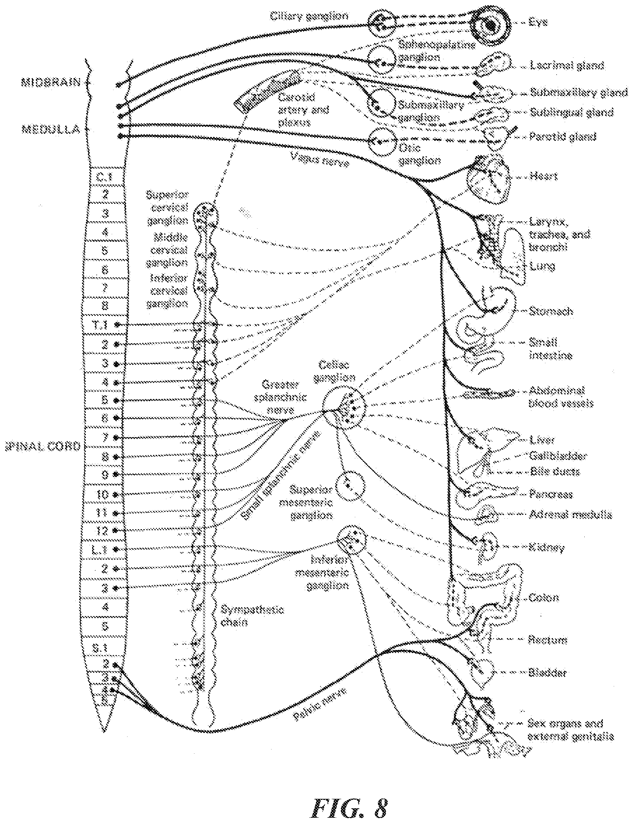

As shown in FIG. 8, the SNS provides a network of nerves that allows the brain to communicate with the body. Sympathetic nerves originate inside the vertebral column, toward the middle of the spinal cord in the intermediolateral cell column (or lateral horn), beginning at the first thoracic segment of the spinal cord and are thought to extend to the second or third lumbar segments. Because its cells begin in the thoracic and lumbar regions of the spinal cord, the SNS is said to have a *thoracolumbar outflow.* Axons of these nerves leave the spinal cord through the anterior rootlet/root. They pass near the spinal (sensory) ganglion, where they enter the anterior rami of the spinal nerves. However, unlike somatic innervation, they quickly separate out through white rami connectors which connect to either the paravertebral (which lie near the vertebral column) or prevertebral (which lie near the aortic bifurcation) ganglia extending alongside the spinal column.

In order to reach the target organs and glands, the axons should travel long distances in the body, and, to accomplish this, many axons relay their message to a second cell through synaptic transmission. The ends of the axons link across a space, the synapse, to the dendrites of the second cell. The first cell (the presynaptic cell) sends a neurotransmitter across the synaptic cleft where it activates the second cell (the postsynaptic cell). The message is then carried to the final destination.

In the SNS and other components of the peripheral nervous system, these synapses are made at sites called ganglia. The cell that sends its fiber is called a preganglionic cell, while the cell whose fiber leaves the ganglion is called a postganglionic cell. As mentioned previously, the preganglionic cells of the SNS are located between the first thoracic (T1) segment and third lumbar (L3) segments of the spinal cord. Postganglionic cells have their cell bodies in the ganglia and send their axons to target organs or glands.

The ganglia include not just the sympathetic trunks but also the cervical ganglia (superior, middle and inferior), which sends sympathetic nerve fibers to the head and thorax organs, and the celiac and mesenteric ganglia (which send sympathetic fibers to the gut).

### 2. Innervation of the Kidneys

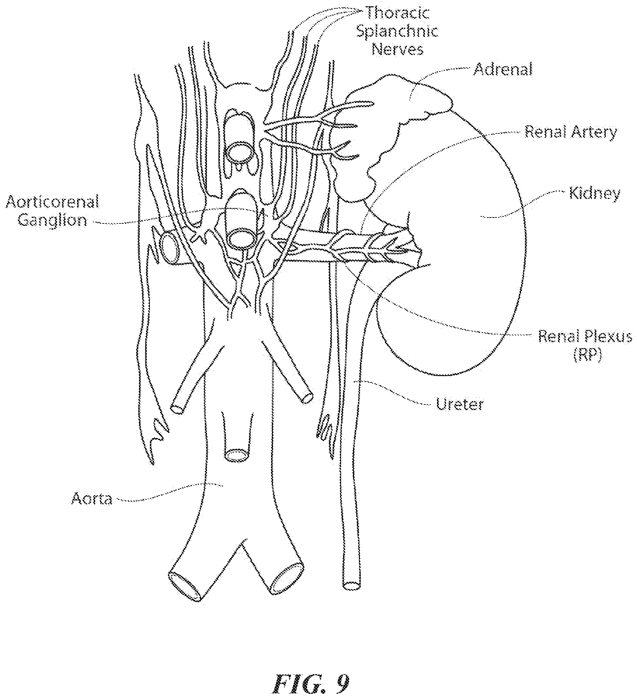

As FIG. 9 shows, the kidney is innervated by the renal plexus RP, which is intimately associated with the renal artery. The renal plexus RP is an autonomic plexus that surrounds the renal artery and is embedded within the adventitia of the renal artery. The renal plexus RP extends along the renal artery until it arrives at the substance of the kidney. Fibers contributing to the renal plexus RP arise from the celiac ganglion, the superior mesenteric ganglion, the aorticorenal ganglion and the aortic plexus. The renal plexus RP, also referred to as the renal nerve, is predominantly comprised of sympathetic components. There is no (or at least very minimal) parasympathetic innervation of the kidney.

Preganglionic neuronal cell bodies are located in the intermediolateral cell column of the spinal cord. Preganglionic axons pass through the paravertebral ganglia (they do not synapse) to become the lesser splanchnic nerve, the least splanchnic nerve, first lumbar splanchnic nerve, second lumbar splanchnic nerve, and travel to the celiac ganglion, the superior mesenteric ganglion, and the aorticorenal ganglion. Postganglionic neuronal cell bodies exit the celiac ganglion, the superior mesenteric ganglion, and the aorticorenal ganglion to the renal plexus RP and are distributed to the renal vasculature.

### 3. Renal Sympathetic Neural Activity

Messages travel through the SNS in a bidirectional flow. Efferent messages may trigger changes in different parts of the body simultaneously. For example, the sympathetic nervous system may accelerate heart rate; widen bronchial passages; decrease motility (movement) of the large intestine; constrict blood vessels; increase peristalsis in the esophagus; cause pupil dilation, piloerection (goose bumps) and perspiration (sweating); and raise blood pressure. Afferent messages carry signals from various organs and sensory receptors in the body to other organs and, particularly, the brain.

Hypertension, heart failure and chronic kidney disease are a few of many disease states that result from chronic activation of the SNS, especially the renal sympathetic nervous system. Chronic activation of the SNS is a maladaptive response that drives the progression of these disease states. Pharmaceutical management of the renin-angiotensin-aldosterone system (RAAS) has been a longstanding, but somewhat ineffective, approach for reducing overactivity of the SNS.

As mentioned above, the renal sympathetic nervous system has been identified as a major contributor to the complex pathophysiology of hypertension, states of volume overload (such as heart failure), and progressive renal disease, both experimentally and in humans. Studies employing radiotracer dilution methodology to measure overflow of norepinephrine from the kidneys to plasma revealed increased renal norepinephrine (NE) spillover rates in patients with essential hypertension, particularly so in young hypertensive subjects, which in concert with increased NE spillover from the heart, is consistent with the hemodynamic profile typically seen in early hypertension and characterized by an increased heart rate, cardiac output, and renovascular resistance. It is now known that essential hypertension is commonly neurogenic, often accompanied by pronounced sympathetic nervous system overactivity.

Activation of cardiorenal sympathetic nerve activity is even more pronounced in heart failure, as demonstrated by an exaggerated increase of NE overflow from the heart and the kidneys to plasma in this patient group. In line with this notion is the recent demonstration of a strong negative predictive value of renal sympathetic activation on all-cause mortality and heart transplantation in patients with congestive heart failure, which is independent of overall sympathetic activity, glomerular filtration rate, and left ventricular ejection fraction. These findings support the notion that treatment regimens that are designed to reduce renal sympathetic stimulation have the potential to improve survival in patients with heart failure.

Both chronic and end stage renal disease are characterized by heightened sympathetic nervous activation. In patients with end stage renal disease, plasma levels of norepinephrine above the median have been demonstrated to be predictive for both all-cause death and death from cardiovascular disease. This is also true for patients suffering from diabetic or contrast nephropathy. There is compelling evidence suggesting that sensory afferent signals originating from the diseased kidneys are major contributors to initiating and sustaining elevated central sympathetic outflow in this patient group; this facilitates the occurrence of the well known adverse consequences of chronic sympathetic over activity, such as hypertension, left ventricular hypertrophy, ventricular arrhythmias, sudden cardiac death, insulin resistance, diabetes, and metabolic syndrome.

### (i) Renal Sympathetic Efferent Activity

Sympathetic nerves to the kidneys terminate in the blood vessels, the juxtaglomerular apparatus and the renal tubules. Stimulation of the renal sympathetic nerves causes increased renin release, increased sodium (Na+) reabsorption, and a reduction of renal blood flow. These components of the neural regulation of renal function are considerably stimulated in disease states characterized by heightened sympathetic tone and clearly contribute to the rise in blood pressure in hypertensive patients. The reduction of renal blood flow and glomerular filtration rate as a result of renal sympathetic efferent stimulation is likely a cornerstone of the loss of renal function in cardio-renal syndrome, which is renal dysfunction as a progressive complication of chronic heart failure, with a clinical course that typically fluctuates with the patient's clinical status and treatment. Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release) and diuretics (intended to counter the renal sympathetic mediated sodium and water retention). However, the current pharmacologic strategies have significant limitations including limited efficacy, compliance issues, side effects and others.

### (ii) Renal Sensory Afferent Nerve Activity

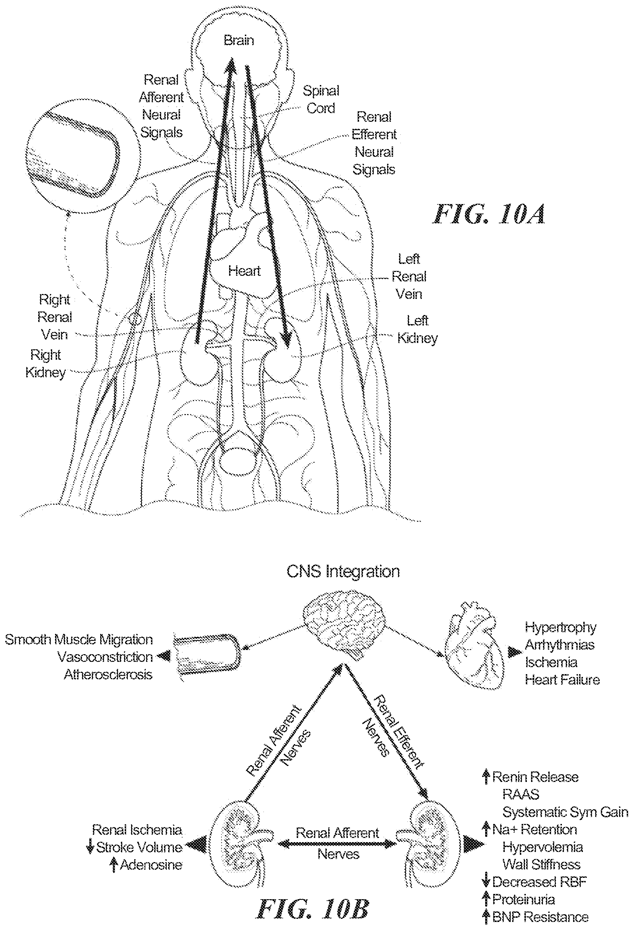

The kidneys communicate with integral structures in the central nervous system via renal sensory afferent nerves. Several forms of "renal injury" may induce activation of sensory afferent signals. For example, renal ischemia, reduction in stroke volume or renal blood flow, or an abundance of adenosine enzyme may trigger activation of afferent neural communication. As shown in FIGS. 10A and 10B, this afferent communication might be from the kidney to the brain or might be from one kidney to the other kidney (via the central nervous system). These afferent signals are centrally integrated and may result in increased sympathetic outflow. This sympathetic drive is directed towards the kidneys, thereby activating the RAAS and inducing increased renin secretion, sodium retention, volume retention and vasoconstriction. Central sympathetic over activity also impacts other organs and bodily structures innervated by sympathetic nerves such as the heart and the peripheral vasculature, resulting in the described adverse effects of sympathetic activation, several aspects of which also contribute to the rise in blood pressure.

The physiology therefore suggests that (i) modulation of tissue with efferent sympathetic nerves will reduce inappropriate renin release, salt retention, and reduction of renal blood flow, and that (ii) modulation of tissue with afferent sensory nerves will reduce the systemic contribution to hypertension and other disease states associated with increased central sympathetic tone through its direct effect on the posterior hypothalamus as well as the contralateral kidney. In addition to the central hypotensive effects of afferent renal denervation, a desirable reduction of central sympathetic outflow to various other sympathetically innervated organs such as the heart and the vasculature is anticipated.

### B. Additional Clinical Benefits of Renal Denervation

As provided above, renal denervation is likely to be valuable in the treatment of several clinical conditions characterized by increased overall and particularly renal sympathetic activity such as hypertension, metabolic syndrome, insulin resistance, diabetes, left ventricular hypertrophy, chronic end stage renal disease, inappropriate fluid retention in heart failure, cardio-renal syndrome, and sudden death. Since the reduction of afferent neural signals contributes to the systemic reduction of sympathetic tone/drive, renal denervation might also be useful in treating other conditions associated with systemic sympathetic hyperactivity. Accordingly, renal denervation may also benefit other organs and bodily structures innervated by sympathetic nerves, including those identified in FIG. 8. For example, as previously discussed, a reduction in central sympathetic drive may reduce the insulin resistance that afflicts people with metabolic syndrome and Type II diabetics. Additionally, patients with osteoporosis are also sympathetically activated and might also benefit from the down regulation of sympathetic drive that accompanies renal denervation.

### C. Achieving Intravascular Access to the Renal Artery

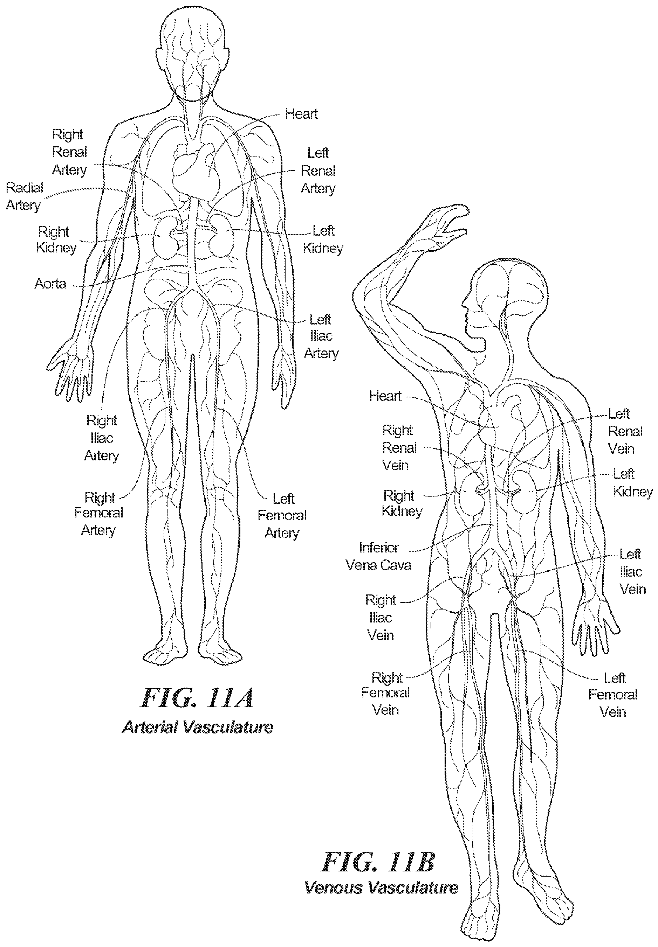

In accordance with the present technology, neuromodulation of a left and/or right renal plexus RP, which is intimately associated with a left and/or right renal artery, may be achieved through intravascular access. As FIG. 11A shows, blood moved by contractions of the heart is conveyed from the left ventricle of the heart by the aorta. The aorta descends through the thorax and branches into the left and right renal arteries. Below the renal arteries, the aorta bifurcates at the left and right iliac arteries. The left and right iliac arteries descend, respectively, through the left and right legs and join the left and right femoral arteries.

As FIG. 11B shows, the blood collects in veins and returns to the heart, through the femoral veins into the iliac veins and into the inferior vena cava. The inferior vena cava branches into the left and right renal veins. Above the renal veins, the inferior vena cava ascends to convey blood into the right atrium of the heart. From the right atrium, the blood is pumped through the right ventricle into the lungs, where it is oxygenated. From the lungs, the oxygenated blood is conveyed into the left atrium. From the left atrium, the oxygenated blood is conveyed by the left ventricle back to the aorta.

As will be described in greater detail later, the femoral artery may be accessed and cannulated at the base of the femoral triangle just inferior to the midpoint of the inguinal ligament. A catheter may be inserted percutaneously into the femoral artery through this access site, passed through the iliac artery and aorta, and placed into either the left or right renal artery. This comprises an intravascular path that offers minimally invasive access to a respective renal artery and/or other renal blood vessels.

The wrist, upper arm, and shoulder region provide other locations for introduction of catheters into the arterial system. For example, catheterization of either the radial, brachial, or axillary artery may be utilized in select cases. Catheters introduced via these access points may be passed through the subclavian artery on the left side (or via the subclavian and brachiocephalic arteries on the right side), through the aortic arch, down the descending aorta and into the renal arteries using standard angiographic technique.

### D. Properties and Characteristics of the Renal Vasculature

Since neuromodulation of a left and/or right renal plexus RP may be achieved in accordance with the present technology through intravascular access, properties and characteristics of the renal vasculature may impose constraints upon and/or inform the design of apparatus, systems, and methods for achieving such renal neuromodulation. Some of these properties and characteristics may vary across the patient population and/or within a specific patient across time, as well as in response to disease states, such as hypertension, chronic kidney disease, vascular disease, end-stage renal disease, insulin resistance, diabetes, metabolic syndrome, etc. These properties and characteristics, as explained herein, may have bearing on the efficacy of the procedure and the specific design of the intravascular device. Properties of interest may include, for example, material/mechanical, spatial, fluid dynamic/hemodynamic and/or thermodynamic properties.

As discussed previously, a catheter may be advanced percutaneously into either the left or right renal artery via a minimally invasive intravascular path. However, minimally invasive renal arterial access may be challenging, for example, because as compared to some other arteries that are routinely accessed using catheters, the renal arteries are often extremely tortuous, may be of relatively small diameter, and/or may be of relatively short length. Furthermore, renal arterial atherosclerosis is common in many patients, particularly those with cardiovascular disease. Renal arterial anatomy also may vary significantly from patient to patient, which further complicates minimally invasive access. Significant inter-patient variation may be seen, for example, in relative tortuosity, diameter, length, and/or atherosclerotic plaque burden, as well as in the take-off angle at which a renal artery branches from the aorta. Apparatus, systems and methods for achieving renal neuromodulation via intravascular access should account for these and other aspects of renal arterial anatomy and its variation across the patient population when minimally invasively accessing a renal artery.

In addition to complicating renal arterial access, specifics of the renal anatomy also complicate establishment of stable contact between neuromodulatory apparatus and a luminal surface or wall of a renal artery. When the neuromodulatory apparatus includes an energy delivery element, such as an electrode, consistent positioning and appropriate contact force applied by the energy delivery element to the vessel wall are important for predictability. However, navigation is impeded by the tight space within a renal artery, as well as tortuosity of the artery. Furthermore, establishing consistent contact is complicated by patient movement, respiration, and/or the cardiac cycle because these factors may cause significant movement of the renal artery relative to the aorta, and the cardiac cycle may transiently distend the renal artery (i.e., cause the wall of the artery to pulse).

Even after accessing a renal artery and facilitating stable contact between neuromodulatory apparatus and a luminal surface of the artery, nerves in and around the adventia of the artery should be safely modulated via the neuromodulatory apparatus. Effectively applying thermal treatment from within a renal artery is non-trivial given the potential clinical complications associated with such treatment. For example, the intima and media of the renal artery are highly vulnerable to thermal injury. As discussed in greater detail below, the intima-media thickness separating the vessel lumen from its adventitia means that target renal nerves may be multiple millimeters distant from the luminal surface of the artery. Sufficient energy should be delivered to or heat removed from the target renal nerves to modulate the target renal nerves without excessively cooling or heating the vessel wall to the extent that the wall is frozen, desiccated, or otherwise potentially affected to an undesirable extent. A potential clinical complication associated with excessive heating is thrombus formation from coagulating blood flowing through the artery. Given that this thrombus may cause a kidney infarct, thereby causing irreversible damage to the kidney, thermal treatment from within the renal artery should be applied carefully. Accordingly, the complex fluid mechanics and thermodynamic conditions present in the renal artery during treatment, particularly those that may impact heat transfer dynamics at the treatment site, may be important in applying energy (e.g., heating thermal energy) and/or removing heat from the tissue (e.g., cooling thermal conditions) from within the renal artery.

The neuromodulatory apparatus should also be configured to allow for adjustable positioning and repositioning of the energy delivery element(s) within the renal artery since location of treatment may also impact clinical efficacy. For example, it may be tempting to apply a full circumferential treatment from within the renal artery given that the renal nerves may be spaced circumferentially around a renal artery. In some situations, full-circle lesion likely resulting from a continuous circumferential treatment may be potentially related to renal artery stenosis. Therefore, the formation of more complex lesions along a longitudinal dimension of the renal artery via the mesh structures described herein and/or repositioning of the neuromodulatory apparatus to multiple treatment locations may be desirable. It should be noted, however, that a benefit of creating a circumferential ablation may outweigh the potential of renal artery stenosis or the risk may be mitigated with certain embodiments or in certain patients and creating a circumferential ablation could be a goal. Additionally, variable positioning and repositioning of the neuromodulatory apparatus may prove to be useful in circumstances where the renal artery is particularly tortuous or where there are proximal branch vessels off the renal artery main vessel, making treatment in certain locations challenging. Manipulation of a device in a renal artery should also consider mechanical injury imposed by the device on the renal artery. Motion of a device in an artery, for example by inserting, manipulating, negotiating bends and so forth, may contribute to dissection, perforation, denuding intima, or disrupting the interior elastic lamina.

Blood flow through a renal artery may be temporarily occluded for a short time with minimal or no complications. However, occlusion for a significant amount of time should be avoided because to prevent injury to the kidney such as ischemia. It could be beneficial to avoid occlusion all together or, if occlusion is beneficial to the embodiment, to limit the duration of occlusion, for example to 2-5 minutes.

Based on the above described challenges of (1) renal artery intervention, (2) consistent and stable placement of the treatment element against the vessel wall, (3) effective application of treatment across the vessel wall, (4) positioning and potentially repositioning the treatment apparatus to allow for multiple treatment locations, and (5) avoiding or limiting duration of blood flow occlusion, various independent and dependent properties of the renal vasculature that may be of interest include, for example, (a) vessel diameter, vessel length, intima-media thickness, coefficient of friction, and tortuosity; (b) distensibility, stiffness and modulus of elasticity of the vessel wall; (c) peak systolic, end-diastolic blood flow velocity, as well as the mean systolic-diastolic peak blood flow velocity, and mean/max volumetric blood flow rate; (d) specific heat capacity of blood and/or of the vessel wall, thermal conductivity of blood and/or of the vessel wall, and/or thermal convectivity of blood flow past a vessel wall treatment site and/or radiative heat transfer; (e) renal artery motion relative to the aorta induced by respiration, patient movement, and/or blood flow pulsatility: and (f) as well as the take-off angle of a renal artery relative to the aorta. These properties will be discussed in greater detail with respect to the renal arteries. However, dependent on the apparatus, systems and methods utilized to achieve renal neuromodulation, such properties of the renal arteries, also may guide and/or constrain design characteristics.

As noted above, an apparatus positioned within a renal artery should conform to the geometry of the artery. Renal artery vessel diameter, D_{RA}, typically is in a range of about 2-10 mm, with most of the patient population having a D_{RA} of about 4mm to about 8mm and an average of about 6 mm. Renal artery vessel length, L_{RA}, between its ostium at the aorta/renal artery juncture and its distal branchings, generally is in a range of about 5-70 mm, and a significant portion of the patient population is in a range of about 20-50mm. Since the target renal plexus is embedded within the adventitia of the renal artery, the composite Intima-Media Thickness, IMT, (i.e., the radial outward distance from the artery's luminal surface to the adventitia containing target neural structures) also is notable and generally is in a range of about 0.5-2.5 mm, with an average of about 1.5 mm. Although a certain depth of treatment is important to reach the target neural fibers, the treatment should not be too deep (e.g., > 5 mm from inner wall of the renal artery) to avoid non-target tissue and anatomical structures such as the renal vein.

An additional property of the renal artery that may be of interest is the degree of renal motion relative to the aorta, induced by respiration and/or blood flow pulsatility. A patient's kidney, which located at the distal end of the renal artery, may move as much as 4" cranially with respiratory excursion. This may impart significant motion to the renal artery connecting the aorta and the kidney, thereby requiring from the neuromodulatory apparatus a unique balance of stiffness and flexibility to maintain contact between the thermal treatment element and the vessel wall during cycles of respiration. Furthermore, the take-off angle between the renal artery and the aorta may vary significantly between patients, and also may vary dynamically within a patient, e.g., due to kidney motion. The take-off angle generally may be in a range of about 30°-135°.

### VI. Conclusion

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology, as those skilled in the relevant art will recognize. For example, while steps are presented in a given order, alternative examples may perform steps in a different order. The various embodiments described herein may also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Certain aspects of the present technology may take the form of computer-executable instructions, including routines executed by a controller or other data processor. In some embodiments, a controller or other data processor is specifically programmed, configured, and/or constructed to perform one or more of these computer-executable instructions. Furthermore, some aspects of the present technology may take the form of data (e.g., non-transitory data) stored or distributed on computer-readable media, including magnetic or optically readable and/or removable computer discs as well as media distributed electronically over networks. Accordingly, data structures and transmissions of data particular to aspects of the present technology are encompassed within the scope of the present technology. The present technology also encompasses methods of both programming computer-readable media to perform particular steps and executing the steps.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A system (10), comprising:
an intravascular catheter (12) comprising an elongated shaft (16) having a proximal portion (18) and a distal portion (20), wherein the distal portion (20) comprises a therapeutic assembly comprising multiple energy delivery elements (24) configured to be positioned within a blood vessel of a human patient and at least proximate to neural fibers associated with sympathetic neural function of the patient; and
an energy source configured for connection to the energy delivery elements (24) and configured to deliver energy via the energy delivery elements (24) to the neural fibers; and
wherein the energy source comprises a controller (42) including memory and processing circuitry, the memory storing instructions that, when executed by the controller (42) using the processing circuitry, cause the controller to-
obtain a first data set associated with a first energy delivery element (24) of the multiple energy delivery elements (24), wherein the first data set includes a first impedance parameter and a first temperature parameter;
obtain a second data set associated with a second energy delivery element (24) of the multiple energy delivery elements, wherein the second energy delivery element (24) is spaced apart from the first energy delivery element (24) by an element separation distance, and wherein the second data set includes a second impedance parameter and a second temperature parameter; and
determine a blood flow velocity in the blood vessel at or otherwise proximate to the therapeutic assembly based on the first data set and the second data set.

2. The system (10) of claim 1 wherein the instructions further cause the controller (42) to adjust energy delivery to the energy delivery elements (24) based, at least in part, on the blood flow velocity.

3. The system (10) of claim 1 wherein:
the first data set corresponds to a first contrast event at the first energy delivery element (24); and
the second data set corresponds to a second contrast event at the second energy delivery element (24);
wherein the instructions further cause the controller to-
determine a first time at which a contrast medium breaches the first delivery element (24);
determine a second time at which the contrast medium breaches the second delivery element (24); and
determine the blood flow velocity by dividing the element separation distance by the difference between the first time and the second time.

4. The system (10) of claim 1 wherein the instructions further cause the controller (42) to determine whether the blood flow velocity is within a pre-determined range before initiating energy delivery to the energy delivery elements (24).

## Patentansprüche

1. System (10), das Folgendes umfasst:
einen intravaskulären Katheter (12), der einen länglichen Schaft (16) umfasst, der einen proximalen Abschnitt (18) und einen distalen Abschnitt (20) aufweist, wobei der distale Abschnitt (20) eine therapeutische Anordnung umfasst, die mehrere Energieabgabeelemente (24) umfasst, die konfiguriert sind, um innerhalb eines Blutgefäßes eines menschlichen Patienten und wenigstens nahe an Nervenfasern, die mit einer sympathischen Nervenfunktion des Patienten im Zusammenhang stehen, positioniert zu werden; und
eine Energiequelle, die für eine Verbindung mit den Energieabgabeelementen (24) konfiguriert ist und konfiguriert ist, um Energie über die Energieabgabeelemente (24) an die Nervenfasern abzugeben; und
wobei die Energiequelle eine Steuerung (42) umfasst, die einen Speicher und eine Verarbeitungsschaltung beinhaltet, wobei der Speicher Anweisungen speichert, die, wenn sie durch die Steuerung (42) unter Verwendung der Verarbeitungsschaltung ausgeführt werden, die Steuerung zu Folgendem veranlassen:
Erhalten eines ersten Datensatzes, der mit einem ersten Energieabgabeelement (24) der mehreren Energieabgabeelemente (24) im Zusammenhang steht, wobei der erste Datensatz einen ersten Impedanzparameter und einen ersten Temperaturparameter beinhaltet;
Erhalten eines zweiten Datensatzes, der mit einem zweiten Energieabgabeelement (24) der mehreren Energieabgabeelemente im Zusammenhang steht, wobei das zweite Energieabgabeelement (24) von dem ersten Energieabgabeelement (24) durch eine Elementtrennungsdistanz beabstandet ist, und wobei der zweite Datensatz einen zweiten Impedanzparameter und einen zweiten Temperaturparameter beinhaltet; und
Bestimmen einer Blutflussgeschwindigkeit in dem Blutgefäß an oder andernfalls nahe der therapeutischen Anordnung basierend auf dem ersten Datensatz und dem zweiten Datensatz.

2. System (10) nach Anspruch 1, wobei die Anweisungen die Steuerung (42) ferner veranlassen, die Energieabgabe an die Energieabgabeelemente (24) wenigstens teilweise basierend auf der Blutflussgeschwindigkeit anpasst.

3. System (10) nach Anspruch 1, wobei:
der erste Datensatz einem ersten Kontrastereignis an dem ersten Energieabgabeelement (24) entspricht; und
der zweite Datensatz einem zweiten Kontrastereignis an dem zweiten Energieabgabeelement (24) entspricht;
wobei die Anweisungen die Steuerung ferner zu Folgendem veranlassen:
Bestimmen einer ersten Zeit zu der ein Kontrastmittel das erste Abgabeelement (24) durchbricht;
Bestimmen einer zweiten Zeit zu der das Kontrastmittel das zweite Abgabeelement (24) durchbricht; und
Bestimmen der Blutflussgeschwindigkeit durch Teilen des Elementtrennungsabstands durch die Differenz zwischen der ersten Zeit und der zweiten Zeit.

4. System (10) nach Anspruch 1, wobei die Anweisungen die Steuerung (42) ferner veranlassen, zu bestimmen, ob die Blutflussgeschwindigkeit innerhalb eines zuvor bestimmten Bereichs liegt, bevor die Energieabgabe an die Energieabgabeelemente (24) eingeleitet wird.

## Revendications

1. Système (10) comprenant:
un cathéter intravasculaire (12) comprenant un arbre allongé (16) présentant une partie proximale (18) et une partie distale (20), dans lequel la partie distale (20) comprend un ensemble thérapeutique comprenant de multiples éléments de distribution d'énergie (24) conçu pour être positionné à l'intérieur d'un vaisseau sanguin d'un patient humain et au moins à proximité des fibres neurales associées à la fonction neurale sympathique du patient ; et
une source d'énergie conçue pour être reliée aux éléments de distribution d'énergie (24) et conçue pour distribuer de l'énergie par l'intermédiaire des éléments de distribution d'énergie (24) aux fibres neurales ; et
dans lequel la source d'énergie comprend un contrôleur (42) comportant une mémoire et des circuits de traitement, la mémoire stockant des instructions qui, lorsqu'elles sont exécutées par le contrôleur (42) à l'aide des circuits de traitement, amènent le contrôleur à
obtenir un premier ensemble de données associé à un premier élément de distribution d'énergie (24) des multiples éléments de distribution d'énergie (24), dans lequel le premier ensemble de données comporte un premier paramètre d'impédance et un premier paramètre de température ;
obtenir un second ensemble de données associé à un second élément de distribution d'énergie (24) des multiples éléments de distribution d'énergie, dans lequel le second élément de distribution d'énergie (24) est espacé du premier élément de distribution d'énergie (24) d'une distance de séparation d'élément, et dans lequel le second ensemble de données comporte un second paramètre d'impédance et un second paramètre de température ; et à
déterminer une vitesse de flux sanguin dans le vaisseau sanguin au niveau ou à proximité de l'ensemble thérapeutique sur la base du premier ensemble de données et du second ensemble de données.

2. Système (10) selon la revendication 1, dans lequel les instructions amènent en outre le contrôleur (42) à régler la distribution d'énergie aux éléments de distribution d'énergie (24) sur la base, au moins en partie, de la vitesse du flux sanguin.

3. Système (10) selon la revendication 1, dans lequel :
le premier ensemble de données correspond à un premier événement de contraste au niveau du premier élément de distribution d'énergie (24) ; et
le second ensemble de données correspond à un second événement de contraste au niveau du second élément de distribution d'énergie (24) ;
dans lequel les instructions amènent en outre le contrôleur à
déterminer un premier moment auquel un produit de contraste enfreint le premier élément de distribution (24) ;
déterminer un second moment auquel le produit de contraste entre en rupture avec le second élément de distribution (24) ; et à
déterminer la vitesse du flux sanguin en divisant la distance de séparation des éléments par la différence entre la première fois et la seconde fois.

4. Système (10) selon la revendication 1, dans lequel les instructions amènent en outre le contrôleur (42) à déterminer si la vitesse du flux sanguin est à l'intérieur d'une plage prédéterminée avant d'initier la distribution d'énergie aux éléments de distribution d'énergie (24).
